# FASCICULE DE BREVET EUROPEEN

(11) **EP 3 433 598 B1**
(45) Date de publication et mention de la délivrance du brevet: **18.09.2024**
(21) Numéro de dépôt: 17713257.8
(22) Date de dépôt: 23.03.2017
(51) Int. Cl.: G01N 1/28, H01J 49/00, C07K 17/10, G01N 30/72, G01N 33/68

(54) **BILLES SOLUBLES POUR LA PRÉPARATION DE SOLUTIONS**
LÖSLICHE KUGELN ZUR HERSTELLUNG VON LÖSUNGEN
SOLUBLE BALLS FOR PREPARING SOLUTIONS

(30) Priorité: 23.03.2016 FR 1652478
(43) Date de publication de la demande: 30.01.2019
(73) Titulaire: Anaquant, 69100 Villeurbanne (FR)
(72) Inventeur: FORTIN, Tanguy, 69006 LYON (FR); BARDET, Chloé, 69007 Lyon (FR); BIARC, Jordane, 69004 LYON (FR)
(74) Mandataire: Icosa
(86) Numéro de dépôt international: PCT/EP2017/056949
(87) Numéro de publication internationale: WO 2017/162801

(56) Documents cités:
- WO-A1-2013/043388
- WO-A2-03/008547
- US-A- 5 591 473
- US-A1- 2008 090 295
- US-B2- 8 216 793
- THOMAS CHANDY ET AL: "Chitosan/polyethylene glycol-alginate microcapsules for oral delivery of hirudin", JOURNAL OF APPLIED POLYMER SCIENCE, vol. 70, no. 11, 12 December 1998 (1998-12-12), US, pages 2143 - 2153, XP055733070, ISSN: 0021-8995, DOI: 10.1002/(SICI)1097-4628(19981212)70:11<2143::AID-APP7>3.0.CO;2-L
- YU HAOFENG ET AL: "Synthesis and In Vitro Sorption Properties of PAA-grafted Cellulose Beads for Selective Binding of LDL", ARTIFICIAL CELLS, BLOOD SUBSTITUTES, AND IMMOBILIZATION BIOTECHNOLOGY., vol. 34, no. 5, 1 January 2006 (2006-01-01), US, pages 501 - 513, XP055871430, ISSN: 1073-1199, DOI: 10.1080/10731190600862795

## Description

### DOMAINE DE L'INVENTION

La présente invention concerne la préparation de solutions, notamment pour la mise en oeuvre de méthodes d'analyse, en particulier par spectrométrie, notamment pour la réalisation de solutions standard utiles par exemple à la calibration d'appareils de spectrométrie ou pour la mise en oeuvre de méthodes de diagnostique. Elle permet la mise en oeuvre d'un procédé aisé de préparation de telles solutions standard.

### ETAT DE LA TECHNIQUE

L'utilisation d'appareils de spectrométrie nécessite une calibration de ces derniers, tant pour s'assurer de son bon fonctionnement, de la qualité des résultats, que pour mettre en oeuvre des méthodes de spectrométrie quantitative.

On connaît par exemple des méthodes d'analyse quantitatives de protéines par spectrométrie de masse au moyen de gammes de produits marqués nécessitant la réalisation de solutions standard (EP 1 456 227, WO 2010/035504, WO 2014/066284). La réalisation de telles solutions standard est souvent chronophage et nécessite une attention toute particulière pour s'assurer une bonne reproductibilité des résultats dans le temps et une bonne stabilité.

De même, la préparation de solutions de réactifs pour des réactions chimiques ou enzymatiques en quantité prédéterminées pourrait être simplifiée si on disposait de moyens simples de préparation de solutions standard qui préservent l'activité de ces réactifs, molécules chimiques ou enzymes.

Une méthode d'encapsulation de protéines est décrit par Chandy et al., (Journal of Applied Polymer Science, 1998, 70, pages 2143-2153).

La demande WO 2013/043388 décrit la préparation par lyophilisation de particules constituées essentiellement de protéines à dissoudre avec du sel, un tampon et un agent de charge. Cependant, cette technique de lyophilisation ne permet pas de réaliser des particules calibrées susceptibles d'être employées pour la préparation de solutions standard.

Le brevet US 5,591,473 décrit la préparation d'un complexe protéine-polysaccharide, bien connu de l'homme du métier, mais ne décrit pas la préparation de tels complexes pour la préparation billes calibrées.

La présente invention permet de résoudre ce problème technique avec des billes solubles supportant au moins dix produits différents à solubiliser greffés à leur surface.

### EXPOSE DE L'INVENTION

L'invention concerne un procédé de préparation d'une solution d'au moins dix produits différents à dissoudre selon la revendication 1.

Elle concerne aussi un ensemble de billes solubles supportant au moins dix produits différents à analyser selon la revendication 7.

L'invention concerne également une gamme de produits à analyser selon la revendication 9.

### DESCRIPTION DETAILLEE DE L'INVENTION

La présente invention concerne un procédé de préparation d'une solution d'au moins dix produits différents, caractérisé en ce qu'il comprend la dissolution d'une ou plusieurs billes solubles dans un solvant de la solution à la surface desquelles sont greffés lesdits produits.

Par bille, que l'on peut également appeler grain, on entend selon l'invention tout support solide particulaire de forme patatoïde susceptible de s'écouler au travers d'un tamis. Toutefois, l'objectif étant de réaliser des solutions standard, l'homme du métier choisira de préférence des billes de forme homogène, notamment ovoïdes, de préférence essentiellement rondes.

Les billes sont calibrées avec un diamètre homogène, de 1 à 4 mm. La mesure du diamètre des billes est bien connue de l'homme du métier. S'agissant de préférence de billes produites industriellement, le diamètre moyen d'un ensemble de bille produites dans un même lot est généralement homogène, avec une marge d'erreur de moins de 5%, de préférence de moins de 3%. Le diamètre des billes peut contrôlé par l'utilisation de tamis tolérant une marge d'erreur de 5%, voire de 3%, de la taille attendue.

L'homme du métier connaît bien les constituants des billes et saura les choisir en fonction du solvant employé dans les solutions et de l'usage de la solution obtenue, notamment de spectrométrie, et pour un produit à analyser, de la nature du produit et de la méthode de spectrométrie. En effet, les constituants des billes ne doivent pas interférer avec les produits greffés après dissolution, et notamment avec les appareils de spectrométrie et les résultats obtenus.

Par « greffé », on entend selon l'invention toute interaction entre le support et le produit qui permet de lier le produit à son support lors de son stockage (produit sec) et facilite la libération du produit après dissolution du support. Cette liaison peut être une liaison physico-chimique forte ou faible, en particulier une adsorption du produit à la surface du support. Il est important que la liaison produit/support soit rompue de sorte que le produit soit substantiellement intégralement libéré dans la solution. L'homme du métier saura déterminer cette libération intégrale en comparant la concentration attendue en produit dans la solution à la concentration mesurée.

Le solvant des solutions est généralement de l'eau ou une solution hydroacloolique, de préférence de l'eau.

La bille est avantageusement constituée de un ou plusieurs composés hydrosolubles choisis parmi les sucres, les polyosides, les acides hydroxycarboxyliques, et leurs mélanges, en particulier choisis parmi le saccharose, le lactose, les alginates, l'acide lactique et leurs mélanges.

On citera en particulier les billes employées comme support pour des préparations homéopathiques, bien connues de l'homme du métier, notamment décrites dans WO 98/07411.

De manière avantageuse, la quantité de produit greffé sur chaque bille est prédéterminée de sorte que chaque bille d'un ensemble de billes comprend substantiellement la même quantité de produit, permettant ainsi à l'homme du métier de connaître la quantité de produit dans la solution par simple multiplication du nombre de billes dissoutes dans la solution.

La quantité de produit va de 1 picogramme à 100 microgrammes par bille.

Le procédé selon l'invention est particulièrement adapté pour préparer des solutions de produits à analyser, plus particulièrement par spectroscopie.

Bien entendu, le procédé selon l'invention est adapté pour la préparation aisée de solutions de produits de concentrations prédéterminées, quelque soit la finalité de ces solutions.

Par produit à dissoudre, on entend tout produit dont la dissolution permet de réaliser des solutions de concentrations prédéterminées, comme des molécules chimiques, des peptides ou des protéines, notamment des enzymes.

Selon un mode particulier de réalisation, l'invention concerne un procédé de préparation d'une solution d'au moins dix produits différents pour son analyse par spectrométrie, caractérisé en ce qu'il comprend la dissolution d'une ou plusieurs billes solubles dans un solvant de la solution à la surface desquelles sont greffés les dits produits à analyser

Selon un autre mode particulier de réalisation, l'invention concerne un procédé de préparation d'une solution d'au moins dix enzymes actives différentes, caractérisé en ce qu'il comprend la dissolution d'une ou plusieurs billes solubles dans le solvant de la solution à la surface desquelles sont greffés les enzymes dont l'activité à été préservée.

L'homme du métier connaît les différents produits susceptibles d'être employés pour préparer des solutions de concentrations prédéterminées, en particulier pour des analyses par spectrométrie, notamment comme produits de calibration, ou pour préparer des solutions d'enzymes. On citera notamment les peptides, protéines ou petites molécules. Les molécules greffées peuvent être marquées isotopiquement, deutérées ou contenir des modifications comme les phosphorylations, les oxydations, les carbamidométhylation ou tout autre modification post traductionnelle connues dans le cas des peptides ou des protéines. Les molécules greffées peuvent être synthétisées chimiquement puis adsorbé sur le support polymérique purifiées ou non. Les molécules peuvent également être purifiées directement à partir d'échantillon biologique avant d'être greffées sur les supports polymériques ou être ajouté greffée lors de la production par couche successive des supports polymériques.

La bille selon l'invention peut comprendre plusieurs produits greffés, chacun dans des proportions prédéterminées, indépendantes les unes des autres. Une même bielle peut avoir greffé à sa surface 10, 20 voir 30 produits différents ou plus. Une bille greffée avec plus de 70 produits différents a été préparée selon la présente invention.

Parmi les produits susceptibles d'être greffés, seuls ou combinés, on peut citer des peptides ou des petites molécules organiques, notamment des produits ayant une activité thérapeutique, comme des antibiotiques, en particulier les produits ci-après

| **Sequence peptide/ Nom molecule** | **Sequence peptide/ Nom molecule** | **Sequence peptide/ Nom molecule** | **Sequence peptide/ Nom molecule** | **Sequence peptide/ Nom molecule** |
|---|---|---|---|---|
| EGVLYVGSK | Bitertanol | TFDPYYAVALVK | Isoprocarb | VQIINK |
| EGVVAAAEK | Boscalid | FTDPVNIISVYK | Isoproturon | VQIVY[Pho]KP VDLSK |
| EGVVHGVTTVAE K | Bromucanozole | LGDPLGADVAQV TGALR | Ivermectin | AVFVDLEPTVID EVR |
| EQVTNVGGAVV TGVTAVAQK | Bupirimate | GIDPEAVLADLR | Kresoxim-methyl | VGINYQPPTVVPG GDLAK |
| QGVAEAAGK | Buprofezin | YDPLVVFSHGLGA FR | Linuron | VAPEEHPVLLTEA PLNPK |
| TVEGAGNIAAA TGFVK | Butafenacil | QDPNEILIFWSK | Lufenuron | GYSFTTTAER |
| AEEAGIGDTPN QEDQAAGHVT[ Pho]QAR | Butocarboxim | EQDPVTNVGGAVV TGVTAVAQK | Mandipropamid | DVNAAIAAIK |
| AEEAGIGDTPN QEDQAAGHVTQ AR | Butoxycarboxi m | IDPGSSDGSSSR | Mefenacet | EIIDPVLDR |
| AEEAGIGDTPS LEDEAAGHVT[ Pho]QAR | Carbaryl | EGDPWAAAEK | Mepanipyrim | DSYVGDEAQSK |
| AEEAGIGDTPS LEDEAAGHVTQ AR | Carbendazim | EADPNYIGSDK | Mepronil | AVFPSIVGRPR |
| C[CAM]GSLGN IHHKPGGGQVE VK | Carbetamide | SQDPTVLQNTGGR | Mesotrione | GIAPLQLGK |
| DRTGNDEK | Carbofuran | SGDPGVCLNCR | Metaflumizone | LHTFGDENEDDSE LAR |
| ES[Pho]PLQT PTEDGSEEPGS ETSDAK | Carboxin | ATDPFNPAQDK | Metalaxyl | SGGVCLNCR |
| ES[Pho]PPQP PADDGAEEPGS ETSDAK | Carfentrazone-ethyl | ANDPIGATLNR | Metconazole | SPIAPCIK |
| ESPLQTPTEDG SEEPGSETSDA K | Chlorantranilipr ole | LYYDPSQDNDR | Methabenzthiaz uron | SSLVIQWR |
| ESPPQPPADDG AEEPGSETSDA K | Chlorfluazuron | SDPIAPCIK | Methamidopho s | gentamicine |
| GAAPPGQK | Chlorotoluron | SLADPAWTGK | Methiocarb | FDPVHAALVWGPE K |
| GAASPAQK | Chloroxuron | TLDPFSR | Methomyl | SYDPYWIGIR |
| HLSNVSSTGSI DMVDSPQLATL ADEVSASLAK | Clethodim | LHDPTFGDENEDD SELAR | Methoprotryne | Flusilazole |
| HVLGGGSVQIV YKPVDLSK | Clofentezine | ASDPYLDCIK | Methoxyfenozid e | Flutolanil |
| HVPGGGSVQIV YKPVDLSK | Clothianidin | IGDPSLDNITHVP GGGNK | Metobromuron | Flutriafol |
| IATPRGAAPPG QK | Cyazofamid | ADPAATVVDVDEV R | Metribuzin | Forchlorfenuron |
| [?] IATPRGAASPA QK | Cycluron | VDPSVYPLDR | Mevinphos | Formetanate HCL |
| IGS[Pho]LDN ITHVPGGGNK | Cymoxanil | EGDPYYGYTGAFR | Mexacarbate | Fuberidazole |
| IGS[Pho]TEN LK | Cyproconazole | DSDPAYGFFK | Monocrotophos | Furalaxyl |
| IGSLDNITHVP GGGNK | Cyprodinil | VEDPSWILR | Monolinuron | Furathiocarb |
| IGSTENLK | Cyromazine | SSDPLVIQWR | Moxidectin | Halofenozide |
| LDLSNVQS[Ph o]K | Desmedipham | CIPDFVNAAFGK | Myclobutanil | Hexaconazole |
| LDLSNVQSK | Diclobutrazol | Tacrolimus | Neburon | Hexaflumuron |
| LQTAPVPMPDL K | Dicrotophos | Sirolimus | Nitenpyram | Hexythiazox |
| QEFDTMEDHAG DYTLLQDQEGD MDHGLK QEEEVM[?] | Diethofencarb | Everolimus | Novaluron | Hydramethylnon |
| QEFEVM[Oxi] EDHAGTYGLGD R | Difenoconazole | TCTTPAQGNSMFP SCCCTKPTDGNCT CIPIPSSWAFAK | Nuarimol | Imidacloprid |
| QEFEVMEDHAG TYGLGDR | Diflubenzuron | TCTTPAQGNSMFP SCCCTKPTD | Omethoate | Indoxacarb |
| S[Pho]PVVSG DTS[Pho]PR | Dimethoate | GNCTCIPIPSSWA FAK | Oxadixyl | Ipconazole |
| S[Pho]PVVSG DTSPR | Dimethomorph | KTCTTPAQGNSMF PSCCCT | Oxamyl | Iprovalicarb |
| S[Pho]TPT[P ho]AEDVTAPL VDEGAPGK | Dimoxystrobin | KPTDGNCTCIPIP SSWAFA | Paclobutrazol | Isocarbophos |
| S[Pho]TPT[P ho]AEDVTAPL VDER | Diniconazole | TCTTPAQGNSMFP SCCCTK | Penconazole | GIAPLQLGK |
| S[Pho]TPTAE DVTAPLVDEGA PGK | Dinotefuran | PTDGNCTCIPIPS SWAFAK | Pencycuron | IAKPNVSASTQAS R |
| S[Pho]TPTAE DVTAPLVDER | Dioxacarb | LGPLV*EQGR | Phenmedipham | TLDFHDSNVK |
| SGYSSPGS[Ph o]PGT[Pho]P GSR | Diuron | LGATQSNEITIPV TFESR | Picoxystrobin | VLPEDGPR |
| SGYSSPGS[Ph o]PGTPGSR | Doramectin | NGVPQEESLEDSD VDADFK | Piperonyl butoxide | YLGADYIK |
| SGYSSPGSPGT [Pho]PGSR | Emamectin-benzoate | LGASVPGSQTVVV K | Pirimicarb | 3-Hydroxycarbofur an |
| SGYSSPGSPGT PGSR | Epoxiconazole | NGISQVLEK | Prochloraz | Abamectin |
| SPVVSGDTS[P ho]PR | Eprinomectin | NGFSIQVR | Promecarb | Acephate |
| SPVVSGDTSPR | Etaconazole | DGVTLQK | Prometon | Acetamiprid |
| STPT[Pho]AE DVTAPLVDEGA PGK | Ethiofencarb | SGIPDNAFQSFGR | Propamocarb | Acibenzolar-S-methyl |
| STPT[Pho]AE DVTAPLVDER | Ethiprole | LGLIFDTSNLQSG VPSR | Propargite | Alanycarb |
| STPTAEDVTAP LVDEGAPGK | Ethirimol | LGPQTLSR | Propham | Aldicarb |
| STPTAEDVTAP LVDER | Ethofumesate | EGQLTPLIK | Propiconazole | Aldicarb sulfone |
| T[Pho]PPGS[ Pho]GEPPK | Etoxazole | EGFDDLPLAEQR | Propoxur | Aldicarb sulfoxide |
| T[Pho]PPGSG EPPK | Famoxadone | TGQSSLVPALTDF VR | Prothioconazol e | Ametryn |
| T[Pho]PPSS[ Pho]GEPPK | Fenamidone | GGFPEFVNELHNN GQK | Pymetrozine | Aminocarb |
| T[Pho]PPSSG EPPK | Fenarimol | EGAQLPVIENK | Pyracarbolid | Amitraz |
| T[Pho]PS[Ph o]LPTPPTR | Fenazaquin | AGIPNNQVLGK | Pyraclostrobin | Azoxystrobin |
| TDHGAEIVY[P ho]K | Fenbuconazole | ALVQIVGK | Pyridaben | Tebufenpyrad |
| TDHGAEIVYK | Fenhexamid | GHSGLQPGR | Pyrimethanil | Tebuthiuron |
| TPPGS[Pho]G EPPK | Fenobucarb | ASTPGAAAQIQEV GK | Pyriproxyfen | Teflubenzuron |
| TPPGSGEPPK | Fenoxycarb | WNPTQGK | Quinoxyfen | Temephos |
| TPPSS[Pho]G EPPK | Fenpropimorph | IPLENLQIIGR | Rotenone | Terbumeton |
| TPPSSGEPPK | Fenpyroximate | NLAVQAQGK | Secbumeton | Terbutryn |
| TPS[Pho]LPT PPTR | Fenuron | VLDALQAIGK | Siduron | Tetraconazole |
| TPSLPTPPTR | Fipronil | VTEPISAESGEQV EGR | Simetryn | Thiabendazole |
| VAVVRT[Pho] PPK | Flonicamid | ISPNTSQQNFVTQ GR | Spinetoram | Thiacloprid |
| VAVVRTPPK | Fluazinam | FQAFANGSLLIPD FGGK | Spinosad | Thiamethoxam |
| VQIVYKPVDLS K | Flubendimide | EPISVSSEQVLGK | Spirodiclofen | Thidiazuron |
| HLSNVSS[Pho ]TGSIDMVDSP QLATLADEVSA SLAK | Fludioxonil | AVFQANQENLPIL GK | Spiromesifen | Thiobencarb |
| HVPGGGSVQIV Y[Pho]KPVDL SK | Flufenacet | VQPVSEILQLGK | Spirotetramat | Thiofanox |
| HVSGGGSVQIV YKPVDLSK | Flufenoxuron | ALDVIQAGGK | Spiroxamine | Thiophanate-methyl |
| SGYSS[Pho]P GSPGTPGSR | Fluometuron | IIHESGAQILGR | Sulfentrazone | Triadimefon |
| T[Pho]PSLPT PPTR | Fluoxastrobin | EQLSSVSSFER | Tebuconazole | Triadimenol |
| TPSLPT[Pho] PPTR | Fluquinconazol e | GDVAFVK | Tebufenozide | Trichlorfon |

Parmi les produits susceptibles d'être greffés, seuls ou combinés, on peut citer des protéines, en particulier des enzymes comme la trypsine, la chymotripsine, la papaïne, la pepsine ou toutes autres peptidases. Toute protéine dont l'homme du métier souhaite préparer des solutions de quantité prédéterminée sans dénaturation entre dans le champ de la présente invention.

La préparation de telles billes et les méthodes pour leur imprégnation par des réactifs sont bien connues de l'homme du métier, en particulier en adaptant les méthodes usuelles de préparations de billes pour médicaments homéopathiques (FR 2 574 659, WO 98/07411).

Les billes sont déposées dans un contenant en verre de taille proportionnelle au diamètre des billes. Avantageusement, le volume du contenant doit être 5 fois supérieur au volume totale représenté par les billes, soit, par exemple, 50 billes de 3mm dans un contenant de 4 cm de diamètre.

La solution mère des produits, molécules, à greffer est composée à plus de 70% de solvant organique comme le méthanol ou l'acétonitrile. Les billes sont mises en contact avec la solution mère avec une proportion de 1g de bille pour 100 µl de solution mère pouvant contenir de l'acide afin de favoriser l'adsorption des molécules sur les billes de polymères.

Le procédé de greffage est réalisé de préférence sous agitation permanente afin de garantir une adsorption homogène des molécules sur l'ensemble des billes d'un lot de production. Le procédé de fabrication peut être décomposé en deux étapes, une première étape consistant en l'imprégnation des billes de polymères par une solution contenant les molécules à greffer, puis une seconde étape de séchage des billes de polymères.

L'imprégnation est réalisée de préférence sous agitation dans un contenant en verre, en particulier à une pression allant de 300mbar à 800mbar, de préférence environ 600 mbar et à une température allant de 20°C à 60°C, de préférence environ 40°C, pour une durée comprise entre 10 et 40 minutes, de préférence environ 20 minutes.

Le séchage des billes après imprégnation est réalisé de préférence sous une atmosphère contrôlée à une pression allant de 300mbar à 800mbar, de préférence environ 600 mbar, à une température allant de 20°C à 60°C, de préférence environ 40 °C, avantageusement par un flux de gaz, pouvant être de l'air comprimé, de l'azote ou tout autre gaz inerte, par exemple entrant par l'intermédiaire d'un tube dont l'extrémité est à l'entrée du flacon en verre contenant les billes, pour une durée comprise avantageusement entre 10 et 40 minutes, de préférence environ 10 minutes.

Ce cycle d'imprégnation et séchage peut être répété plusieurs fois, avantageusement à au moins trois reprises pour garantir une homogénéité du greffage des molécules sur les billes de polymère.

Lorsqu'il y a plusieurs produits greffés, on pourra les greffer soit ensembles, à partir d'un solution mère qui les comprend tous, ou encore les greffer en plusieurs groupes successifs, chacun comprenant un ou plusieurs produits.

L'invention concerne aussi un ensemble de billes solubles supportant au moins dix produits différents à analyser greffés à leur surface, telles que décrites précédemment et dans les exemples, caractérisé en ce que les billes ont chacune une quantité prédéterminée de produit adsorbé à leur surface sensiblement égale.

De manière avantageuse, cet ensemble de billes est conditionné dans un emballage qui permet une libération contrôlée du nombre de billes. De tels emballages sont bien connus de l'homme du métier, notamment utilisés pour la libération de billes de compositions homéopathiques, de produits alimentaires tel que les tablettes d'aspartame ou des bonbons.

L'invention concerne aussi une gamme de produits à analyser caractérisée en ce qu'elle comprend au moins deux ensembles de billes selon l'invention, chaque ensemble ayant une quantité prédéterminée de produits greffés différente des autres ensembles.

La différence de quantités de produits greffés entre les différents ensembles dépendra de l'usage de ces ensembles. On pourra choisir des quantités prédéterminées de chaque ensemble séparées par exemple par un facteur 2, ou un facteur 10, ou encore selon une règle logarithmique.

Une telle gamme de produits peut être employée pour la réalisation de gammes d'étalonnage externe utilisées pour la quantification de composés ou la mesure de composé dans des échantillons ayant des niveaux de concentration différents du composé à analyser.

Les billes greffées peuvent avoir différents usages, elles peuvent être utilisées pour la quantification absolue de composés.

Les billes greffées peuvent également être utilisées pour libérer un standard permettant la quantification relative d'échantillon entre eux, les billes seront donc utilisées pour normaliser les résultats issus de l'analyse des échantillons.

Elles peuvent également être utilisées pour libérer des composés utilisés pour le contrôle qualité d'instrument de mesure comme les spectromètres.

Les billes greffées peuvent être utilisées pour libérer des composés utiles à la détection de mécanisme biologique comme des réactions immunitaires, des compétitions entre un anticorps et un antigène ou évaluer la toxicité des composés sur des modèles biologiques, notamment pour des analyses dans le domaine du diagnostique.

De manière avantageuse, les billes pourront être mis dès les premières étapes de la préparation d'échantillon, à une étape intermédiaire de la préparation d'échantillon ou encore à la fin de la préparation d'échantillon avant analyse de l'échantillon final.

### EXEMPLES

### Exemple 1 - Préparations de billes greffées avec des peptides

Une solution mère de peptide est réalisée par la reprise de 200µg de peptide GDVAFVK marqué aux isotope lourd 13C et 15N sur la lysine (K) par 1ml d'une solution de méthanol 0,5% d'acide formique. 100µl de cette solution mère sont prélevé et mis dans 900µl de méthanol pour obtenir une solution à 20µg/ml. 50 billes de saccharose d'un diamètre de 3mm sont déposées dans un ballon en verre d'un volume de 25ml. 100µl de la solution à 20µg/ml sont déposé dans le ballon contenant les billes de saccharose.

Le ballon en verre contenant les billes et la solution peptidique est mis en rotation et sous vide pour atteindre une pression de 600mbar pendant 20 minutes. A l'issue de cette période d'imprégnation, de l'air comprimée est injectée dans le ballon à un débit de 12L/min pendant 10 minutes sous un vide constant de 600mbar. A l'issu de ce premier cycle d'imprégnation/séchage, il est répété deux fois pour obtenir trois cycles complets.

Une bille greffée avec le peptide GDVAFVK est reprise dans 500µl d'une solution 95% H2O, 5% ACN et 0,5% acide formique puis solubilisé pendant 2 minutes.

Un volume de 40µl de l'échantillon obtenu est injecté et analysé selon les conditions suivantes :
- Chaîne chromatographique 1290 de la société Agilent (Les Ulys, France) &
- Colonne Phenomenex Aeris peptide C18, 2,1mm de diamètre interne, 100mm de long, taille de particule de 3,6mm &
- Solvant A : H2O + 0,1% acide formique &
- Solvant B : ACN + 0,1% acide formique
- Gradient de chromatographie liquide suivant :

| min | Flow (ul/min) | Solent A | Solvent B |
|---|---|---|---|
| 0 | 300 | 95 | 5 |
| 1 | 300 | 95 | 5 |
| 8 | 300 | 50 | 50 |
| 8.1 | 300 | 5 | 95 |
| 12 | 300 | 5 | 95 |
| 12.1 | 300 | 95 | 5 |
| 15 | 300 | 95 | 5 |

L'éluat en sortie de colonne chromatographique est directement injecté dans un détecteur à barrette de diode 1290 de la société Agilent. L'absorption en UV est mesurée à une longueur d'onde de 205 nm.

L'éluat en sortie de colonne chromatographique peut également être directement injecté dans la source d'ionisation du spectromètre de masse de type QTRAP^{®} 5500 de la société Sciex (Foster City, Etats Unis d'Amérique). Les paramètres machine utilisés sont les suivants :

| | |
|---|---|
| Type de balayage: | MRM |
| Polarité: | Positive |
| Source d'ionisation: | Turbo V (Sciex) |
| Précurseur: | 372,2 |
| Ion fragment: | 472,3 |
| Ion fragment: | 401,3 |
| Ion fragment: | 571,4 |
| Réglage Q1: | Filtrage avec résolution unitaire |
| Réglage Q3: | Filtrage avec résolution unitaire |
| Tension de cône: | 5500 V |
| Température de source: | 500,00 °C |
| Gaz de nébulisation: | 50,00 psi |
| Gaz chauffant: | 40,00 psi |
| Gaz rideau: | 50,00 psi |

Ce protocole peut également s 'appliquer à d'autres type de molécules comme des pesticides, des antibiotiques ou tout autre petite molécule.

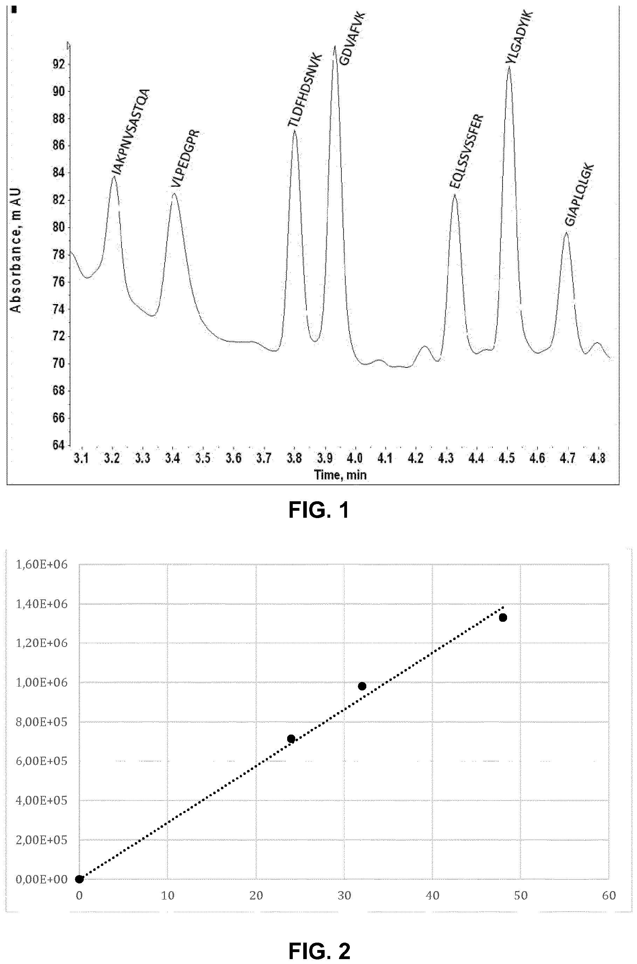

### Exemple 2 - Préparation de billes greffées avec 7 peptides (figure 1)

Les 7 peptides suivant ont été greffés aux billes de saccharose
GIAPLQLG[K(13C6; 15N2)]
EQLSSVSSFE[R(13C6; 15N4)]
TLDFHDSNV[K(13C6; 15N2)]
VLPEDGP[R(13C6; 15N4)]
GDVAFV[K(13C6; 15N2)]
YLGADYI[K(13C6; 15N2)]
IAKPNVSASTQAS[R((13C6) ;15N4)]

Un aliquot de 200µg du premier peptide est dissous dans 1 ml de méthanol. La solution est ajoutée au 200µg du second peptide puis cette dernière est ajoutée au 200µg du troisième peptide et ainsi de suite jusqu'au septième et dernier peptide afin d'obtenir un mélange de 7 peptides avec chacun une concentration de 200µg/ml.

1,8 ml de méthanol est ajouté à la solution mère à 200µg/ml pour obtenir une solution à 70µg/ml. 100µl de cette solution sont ajouté à 250µl de méthanol pour obtenir une solution à 20µg/ml pour chacun des peptides.

Le protocole de greffage décrit dans l'exemple 1 est réalisé pour obtenir des billes greffées avec les 7 peptides.

Une bille greffée avec les 7 peptides est reprise dans 500µl d'une solution 95% H2O, 5% ACN et 0,5% acide formique puis solubilisé pendant 2 minutes.

Un volume de 40µl de l'échantillon obtenu est injecté et analysé selon les conditions suivantes :
- Chaîne chromatographique 1290 de la société Agilent (Les Ulys, France) &
- Colonne Phenomenex Aeris peptide C18, 2,1mm de diamètre interne, 100mm de long, taille de particule de 3,6mm &
- Solvant A : H2O + 0,1% acide formique &
- Solvant B : ACN + 0,1% acide formique
- Gradient de chromatographie liquide suivant :

| min | Flow (ul/min) | Solvant A | Solvant B |
|---|---|---|---|
| 0 | 300 | 95 | 5 |
| 1 | 300 | 95 | 5 |
| 8 | 300 | 50 | 50 |
| 8.1 | 300 | 5 | 95 |
| 12 | 300 | 5 | 95 |
| 12.1 | 300 | 95 | 5 |
| 15 | 300 | 95 | 5 |

L'éluat en sortie de colonne chromatographique est directement injecté dans un détecteur à barrette de diode 1290 de la société Agilent. L'absorption en UV est mesurée à une longueur d'onde de 205 nm. Le résultat est représenté sur la figure 1,

### Exemple 3 - Gamme de Gentamycine (figure 2)

Pour réaliser la solution qui sera déposée sur les billes, la gentamicine 10mg/ml sera diluée 5000 fois. 100µl de la solution mère sont mis dans 900µl d'H2O. 100µl de la solution générée sont mis dans 900µl d'H2O. 500µl de la solution générée sont mis dans 500µl d'H2O pour obtenir une solution à 100µg/ml. 40µl de la solution à 100µg/ml sont mis dans 960µl de méthanol. Le greffage de la gentamicine sur les billes de saccharose de 3mm s'effectue comme décrit dans l'exemple 1.

Une gamme de concentration est réalisée par la reprise d'une bille dans :
- 1ml d'une solution de Methanol/H2O (70%/30%, v/v)
- 750µl d'une solution de Methanol/H2O (70%/30%, v/v)
- 500µl d'une solution de Methanol/H2O (70%/30%, v/v)

Un volume de 20µl de l'échantillon obtenu est injecté et analysé selon les conditions suivantes :
- Chaîne chromatographique 1290 de la société Agilent (Les Ulys, France) &
- Colonne Xbridge Hilic amide 2,1mm*100, 3,5µm colonne de WATERS &
- Solvant A : H2O + 0,1% acide formique &
- Solvant B : ACN + 0,1% acide formique &
- Gradient de chromatographie liquide suivant :

| min | Flow (ul/min) | Solvant A | Solvant B |
|---|---|---|---|
| 0 | 300 | 45 | 55 |
| 1 | 300 | 45 | 55 |
| 4 | 300 | 50 | 15 |
| 4.1 | 300 | 95 | 5 |
| 7 | 300 | 95 | 95 |
| 7.1 | 300 | 35 | 65 |
| 11 | 300 | 35 | 65 |

L'éluat en sortie de colonne chromatographique peut également être directement injecté dans la source d'ionisation du spec- tromètre de masse de type QTRAP^{®} 5500 de la société Sciex (Foster City, Etats Unis d'Amérique). Les paramètres machine utilisés sont les suivants :

| | |
|---|---|
| Type de balayage: | MRM |
| Polarité: | Positive |
| Source d'ionisation: | Turbo V (Sciex) |
| Précurseur: | 478,4 |
| Ion fragment: | 322,4 |
| Ion fragment: | 160,0 |
| Réglage Q1: | Filtrage avec résolution unitaire |
| Réglage Q3: | Filtrage avec résolution unitaire |
| Tension de cône: | 5500 V |
| Température de source: | 500,00 °C |
| Gaz de nébulisation: | 50,00 psi |
| Gaz chauffant: | 40,00 psi |
| Gaz rideau: | 50,00 psi |

Les résultats sont représentés sur la figure 2.

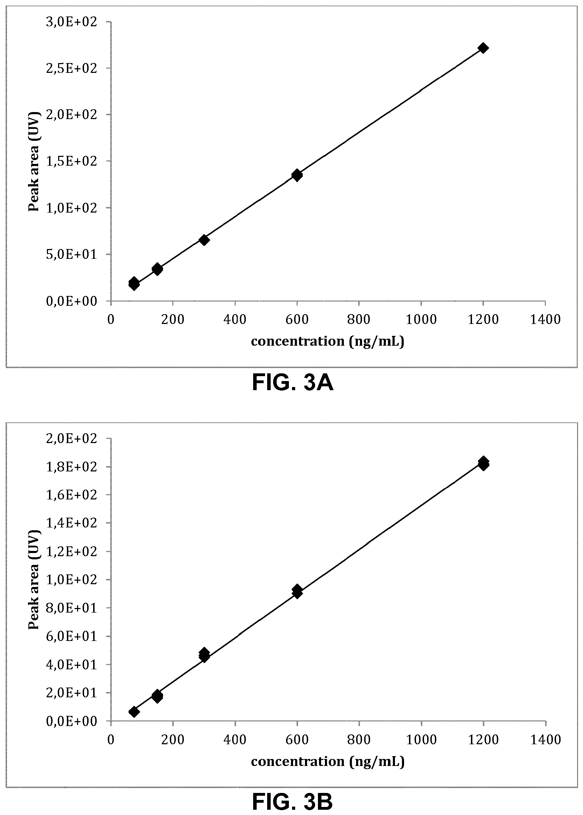

### Exemple 4 Gammes réalisées à deux semaines d'intervalles avec des billes produites à partir de solutions mères de concentration différentes (figures 3A et 3B)

Une solution mère de peptide est réalisée par la reprise de 200µg de peptide GDVAFVK marqué aux isotope lourd 13C et 15N sur la lysine (K) par 1ml d'une solution de méthanol 0,5% d'acide formique. 350µl de la solution mère sont dilué dans 350µl de méthanol 0,5% d'acide formique pour obtenir une solution mère à 100µg/ml. 350µl de la solution mère à 1µg/ml sont dilué dans 350µl de méthanol 0,5% d'acide formique pour obtenir une solution mère à 50µg/ml. 350µl de la solution mère à 50µg/ml sont dilué dans 350µl de méthanol 0,5% d'acide formique pour obtenir une solution mère à 25µg/ml.
350µl de la solution mère à 25µg/ml sont dilué dans 350µl de méthanol 0,5% d'acide formique pour obtenir une solution mère à 12,5µg/ml. 350µl de la solution mère à 12,5µg/ml sont dilué dans 350µl de méthanol 0,5% d'acide formique pour obtenir une solution mère à 6,25µg/ml.

Chacune des solutions mères est utilisé pour le greffage sur des billes de saccharose selon le protocole cité en exemple 1.

Chacune des billes greffées avec des quantités de peptide GDVAFVK allant de 1,2µg à 12,5ng est reprise dans 500µl d'une solution 95% H2O, 5% ACN et 0,5% acide formique puis solubilisé pendant 2 minutes.

Un volume de 40µl de chaque échantillon obtenu est injecté et analysé selon les conditions suivantes :
- Chaîne chromatographique 1290 de la société Agilent (Les Ulys, France)
- Colonne Phenomenex Aeris peptide C18, 2,1mm de diamètre interne, 100mm de long, taille de particule de 3,6mm &
- Solvant A : H2O + 0,1% acide formique &
- Solvant B : ACN + 0,1% acide formique &
- Gradient de chromatographie liquide suivant

| min | Flow (ul/min) | Solvant A | Solvant B |
|---|---|---|---|
| 0 | 300 | 95 | 5 |
| 1 | 300 | 95 | 5 |
| 8 | 300 | 50 | 50 |
| 8.1 | 300 | 5 | 95 |
| 12 | 300 | 5 | 95 |
| 12.1 | 300 | 95 | 5 |
| 15 | 300 | 95 | 5 |

L'éluat en sortie de colonne chromatographique est directement injecté dans un détecteur à barrette de diode 1290 de la société Agilent. L'absorption en UV est mesurée à une longueur d'onde de 205 nm.

Les résultats représentés sur les figures 3A et 3B confirment la reproductibilité de la méthode selon l'invention.

### Exemple 5 - Quantification de l'apolipoprotéine E dans du plasma par le peptide LGPLVEQGR marqué en 13C et 15N sur la valine (V).

Les billes de polymère sont préparées à partir d'une solution mère du peptide LGPLVEQGR à 1µg/ml selon le protocole décrit dans l'exemple 1.

Digestion enzymatique du plasma selon le protocole suivant :
- 1 mL of plasma dilué dans 4mL d'urée à 8M
- 550 µL of DTT à 150mM
- 1700 µL IAA à 150mM
- 30 mL de bicarbonate d'ammonium à 50 mM pH8
- 1 mL de trypsine à 2mg/mL dans 50mM de bicarbonate d'ammonium à pH8.
- 1 mL H2O+0.5%FA sont ajouté à 10 ul de plasma hydrolysé

Une bille de polymère greffé avec 6 ng de peptide LGPLVEQGR est ajoutée

Dessalage et concentration des échantillons par extraction sur phase solide selon le protocole suivant :
- Equilibrage des colonnes HLB Oasis Waters avec lml de méthanol puis 1 ml H₂O/acide formique 0,1%
- Dépôt de l'échantillon qui s'écoule par gravité
- Lavage avec 1ml H₂O/acide formique 0,1%
- Elution avec 1ml de méthanol 80% dans un mélange H₂O/acide formique 0,1%
- Evaporation à sec et reprise dans 200µl d'une solution 95% H2O, 5% ACN et 0,5% acide formique.

Un volume de 20µl de l'échantillon obtenu est injecté et analysé selon les conditions suivantes :
- Chaîne chromatographique 1290 de la société Agilent (Les Ulys, France) 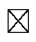
- Colonne Phenomenex Aeris peptide C18, 2,1mm de diamètre interne, 100mm de long, taille de particule de 3,6mm 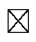
- Solvant A : H2O + 0,1% acide formique 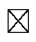
- Solvant B : ACN + 0,1% acide formique 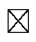
- Gradient de chromatographie liquide suivant

| min | Flow (ul/min) | Solvant A | Solvant B |
|---|---|---|---|
| 0 | 300 | 95 | 5 |
| 1 | 300 | 95 | 5 |
| 8 | 300 | 50 | 50 |
| 8.1 | 300 | 5 | 95 |
| 10 | 300 | 5 | 95 |
| 10.1 | 300 | 95 | 5 |
| 12 | 300 | 95 | 5 |

L'éluat en sortie de colonne chromatographique peut également être directement injecté dans la source d'ionisation du spec- tromètre de masse de type QTRAP^{®} 5500 de la société Sciex (Foster City, Etats Unis d'Amérique). Les paramètres machine utilisés sont les suivants :

| | |
|---|---|
| Type de balayage: | MRM |
| Polarité: | Positive |
| Source d'ionisation: | Turbo V (Sciex) |
| Réglage Q1: | Filtrage avec résolution unitaire |
| Réglage Q3: | Filtrage avec résolution unitaire |
| Tension de cône: | 5500 V |
| Température de source: | 500,00 °C |
| Gaz de nébulisation: | 50,00 psi |
| Gaz chauffant: | 40,00 psi |
| Gaz rideau: | 50,00 psi |

Les valeurs des masses et les paramètres du chemin optique sont les suivants :

| ID | Q1/Q3 | time | DP | EP | CE | CXP |
|---|---|---|---|---|---|---|
| APOEHUMAN_LGPLVEQGR_+ 2y5_light | 484.8 / 855.5 | 100 ms | 66.5 | 10 | 26.3 | 13 |
| APOEHUMAN_LGPLVEQGR_+ 2y6_light | 484.8 / 798.4 | 100 ms | 66.5 | 10 | 26.3 | 13 |
| APOEHUMAN_LGPLVEQGR_+ 2y7_light | 484.8 / 701.4 | 100 ms | 66.5 | 10 | 26.3 | 13 |
| APOEHUMAN_LGPLVEQGR_+ 2y7+2_light | 484.8 / 588.3 | 100 ms | 66.5 | 10 | 26.3 | 13 |
| APOEHUMAN_LGPLVEQGR_+ 2y8_light | 484.8 / 399.7 | 100 ms | 66.5 | 10 | 26.3 | 13 |
| APOEHUMAN_LGPLVEQGR-heavy_+2y5_heavy | 487.8 / 861.5 | 100 ms | 66.5 | 10 | 26.3 | 13 |
| APOEHUMAN_LGPLVEQGR-heavy_+2y6_heavy | 487.8 / 804.5 | 100 ms | 66.5 | 10 | 26.3 | 13 |
| APOEHUMAN_LGPLVEOGR-heavy_+2y7_heavy | 487.8 / 707.4 | 100 ms | 66.5 | 10 | 26.3 | 13 |

### Exemple 6 - Conservation de l'activité enzymatique

Des billes greffées par de la trypsine ont été préparées partir d'une solution mère du de trypsine à 1mg/ml selon le protocole décrit dans l'exemple 1.

L'activité trypsique des protéines après dissolution des billes dans un tampon approprié est observée par l'hydrolyse de l'albumine selon la méthode d'identification des peptides résultants de l'hydrolyse trypsique par spectrométrie de masse. Les peptides issus de l'hydrolyse trypsique sont monitorés spécifiquement afin de déterminer leur présence dans les échantillons.

Les résultats obtenus montrent que l'activité de la trypsine est conservée après le greffage sur les billes, le procédé selon l'invention n'ayant pas d'action de dénaturation de la protéine. Une comparaison avec l'activité de la trypsine non greffée montre une activité similaire, voire supérieure avec la solution de trypsine préparée à partir des billes greffées.

Ces résultats confirment qu'il est possible de préparer des billes de quantités prédéterminées de protéines ou d'enzymes, permettant de faciliter la préparation de solutions dosées avec des protéines, en particulier pour la préparation de solutions d'enzymes.

### REFERENCES

EP 1 456 227,
WO 2010/035504
WO 2014/066284

## Revendications

1. Procédé de préparation d'une solution d'au moins dix produits différents à dissoudre, comprenant la dissolution d'une ou plusieurs billes, chaque bille supportant lesdits produits à dissoudre, les billes étant des billes solubles dans un solvant de la solution, les billes étant calibrées avec un diamètre homogène allant de 1 à 4 mm, et lesdits produits étant greffés à la surface de chacune desdites billes solubles.

2. Procédé selon la revendication 1, **caractérisé en ce que** le solvant de la solution contient de l'eau et la bille est constituée de un ou plusieurs composés hydrosolubles choisis parmi les sucres, les polyosides, les acides hydroxycarboxyliques, et leurs mélanges.

3. Procédé selon la revendication 2, **caractérisé en ce que** le composé hydrosoluble est choisi parmi le saccharose, le lactose, les alginates et l'acide lactique.

4. Procédé selon l'une des revendications 1 à 3, **caractérisé en ce que** la quantité des produits greffés sur chaque bille est prédéterminée, de 1 picogramme à 100 microgrammes par bille.

5. Procédé selon l'une des revendications 1 à 4, **caractérisé en ce que** les produits à dissoudre sont choisis parmi les peptides, les protéines ou petites molécules, éventuellement marquées isotopiquement, deutérées ou pouvant contenir des modifications comme les phosphorylations, les oxydations ou les carbamidométhylations.

6. Procédé selon l'une des revendications 1 à 5, **caractérisé en ce que** la solution préparée est appropriée pour son utilisation dans une méthode d'analyse par spectrométrie.

7. Ensemble de billes solubles supportant au moins dix produits différents à analyser, les dix produits différents étant greffés à la surface de chaque bille, les billes étant calibrées avec un diamètre homogène allant de 1 à 4 mm et ayant chacune une quantité prédéterminée des au moins dix produits greffés à la surface de chaque bille sensiblement égale.

8. Ensemble de billes selon la revendication 7, **caractérisé en ce que** les billes sont conditionnées dans un même emballage permettant une libération contrôlée du nombre de billes.

9. Gamme de produits à analyser **caractérisée en ce qu'**elle comprend au moins deux ensembles de billes selon la revendication 7 ou 8, chaque ensemble ayant une quantité prédéterminée allant de 1 picogramme à 100 microgrammes de produit adsorbé différente des autres ensembles.

10. Gamme selon la revendication 9, **caractérisée en ce que** les quantités prédéterminées allant de 1 picogramme à 100 microgrammes de chaque ensemble sont séparés d'un facteur prédéterminé.

## Patentansprüche

1. Verfahren zur Herstellung einer Lösung von mindestens zehn verschiedenen aufzulösenden Produkten, das das Auflösen einer oder mehrerer Kugeln umfasst, wobei jede Kugel die aufzulösenden Produkte trägt, wobei es sich bei den Kugeln um Kugeln handelt, die in einem Lösungsmittel der Lösung löslich sind, wobei die Kugeln mit einem homogenen Durchmesser von 1 bis 4 mm kalibriert sind und wobei die Produkte auf die Oberfläche jeder löslichen Kugeln aufgepfropft sind.

2. Verfahren nach Anspruch 1, **dadurch gekennzeichnet, dass** das Lösungsmittel der Lösung Wasser enthält und die Kugel aus einer oder mehreren wasserlöslichen Verbindungen besteht, ausgewählt aus Zuckern, Polyosiden, Hydroxycarbonsäuren und deren Mischungen.

3. Verfahren nach Anspruch 2, **dadurch gekennzeichnet, dass** die wasserlösliche Verbindung ausgewählt ist aus Saccharose, Lactose, Alginaten und Milchsäure.

4. Verfahren nach einem der Ansprüche 1 bis 3, **dadurch gekennzeichnet, dass** die Menge der auf jede Kugel aufgepfropften Produkte vorbestimmt ist, von 1 Pikogramm bis 100 Mikrogramm pro Kugel.

5. Verfahren nach einem der Ansprüche 1 bis 4, **dadurch gekennzeichnet, dass** die aufzulösenden Produkte ausgewählt sind aus Peptiden, Proteinen oder kleinen Molekülen, die gegebenenfalls isotopisch markiert, deuteriert sind oder Modifikationen wie Phosphorylierungen, Oxidationen oder Carbamidomethylierungen enthalten können.

6. Verfahren nach einem der Ansprüche 1 bis 5, **dadurch gekennzeichnet, dass** die hergestellte Lösung für die Verwendung in einem spektrometrischen Analyseverfahren geeignet ist.

7. Satz löslicher Kugeln, die mindestens zehn verschiedene zu analysierende Produkte tragen, wobei die zehn verschiedenen Produkte auf die Oberfläche jeder Kugel aufgepfropft sind, wobei die Kugeln mit einem homogenen Durchmesser von 1 bis 4 mm kalibriert sind und jeweils eine vorbestimmte Menge der mindestens zehn auf die Oberfläche jeder Kugel aufgepfropften Produkte aufweisen, die im Wesentlichen gleich ist.

8. Satz Kugeln nach Anspruch 7, **dadurch gekennzeichnet, dass** die Kugeln in derselben Verpackung verpackt sind, die eine kontrollierte Freisetzung der Anzahl von Kugeln ermöglicht.

9. Sortiment zu analysierender Produkte, **dadurch gekennzeichnet, dass** es mindestens zwei Sätze Kugeln nach Anspruch 7 oder 8 umfasst, wobei jeder Satz eine vorbestimmte Menge von 1 Pikogramm bis 100 Mikrogramm adsorbiertes Produkt aufweist, die sich von den anderen Sätzen unterscheidet.

10. Sortiment nach Anspruch 9, **dadurch gekennzeichnet, dass** die vorbestimmten Mengen von 1 Pikogramm bis 100 Mikrogramm jedes Satzes um einen vorbestimmten Faktor getrennt sind.

## Claims

1. Process for preparing a solution of at least ten different products to be dissolved, comprising the dissolution of one or several beads, each bead carrying said products to be dissolved, the beads being soluble beads in a solvent of the solution, the beads being calibrated with a homogenous diameter ranging from 1 to 4 mm, and said products being grafted onto the surface of each of said soluble beads.

2. Process according to claim 1, **characterized in that** the solvent of the solution contains water and the bead consists of one or more water-soluble compounds chosen among sugars, polysaccharides, hydroxycarboxylic acids, and mixtures thereof.

3. Process according to claim 2, **characterized in that** the water-soluble compound is chosen among sucrose, lactose, alginates and lactic acid.

4. Process as according to any one of claims 1 to 3, **characterized in that** the amount of products grafted onto each bead is predetermined, from 1 picogram to 100 micrograms per bead.

5. Process according to any one of claims 1 to 4, **characterized in that** the products to be dissolved are chosen among peptides, proteins or small molecules, eventually isotopically labelled, deuterated or capable of containing modifications such as phosphorylations, oxidations or carbamidomethylations.

6. Process according to any one of claims 1 to 5, **characterized in that** the solution prepared is suitable for its use in a spectrometric analysis method.

7. Set of soluble beads carrying at least ten different products to be analysed, said ten different products being grafted onto the surface of each bead, the beads being calibrated with an homogenous diameter ranging from 1 to 4 mm and each having a predetermined amount of the at least ten products grafted onto the surface of each bead substantially equal.

8. Set of beads according to claim 7, **characterized in that** the beads are packaged in the same packaging allowing controlled release of the number of beads.

9. Range of products to be analysed, **characterized in that** it comprises at least two sets of beads according to claim 7 or 8, each set having a predetermined amount ranging from 1 picogram to 100 micrograms of adsorbed products different from the other sets.

10. Range according to claim 9, **characterized in that** the predetermined amounts ranging from 1 picogram to 100 micrograms of each set are separated by a predetermined factor.
